# EUROPEAN PATENT SPECIFICATION

(11) **EP 1 968 862 B1**
(45) Date of publication and mention of the grant of the patent: **04.08.2010**
(21) Application number: 06828065.0
(22) Date of filing: 22.12.2006
(51) Int. Cl.: B65D 47/42, A61M 35/00, B65D 37/00, A01M 21/04, B65D 41/04, A45D 34/00, B05C 17/00, B65D 51/24, A47K 5/12, B65D 25/38, B65D 51/32

(54) **A LIQUID DISPENSING APPLICATOR WITH FLEXIBLE VALVE MEMBER**
FLÜSSIGKEITSABGABEAUFTRAGSVORRICHTUNG MIT FLEXIBLEM VENTILGLIED
APPLICATEUR DISTRIBUTEUR DE LIQUIDE AVEC ELEMENT DE VALVE SOUPLE

(30) Priority: 23.12.2005 AU 2005907296; 01.06.2006 AU 2006902974
(43) Date of publication of application: 17.09.2008
(73) Proprietor: Withoos, Robert, Keilor, VIC 3036 (AU); Newson, Colin, Niddrie, VIC 3042 (AU)
(72) Inventor: Withoos, Robert, Keilor, VIC 3036 (AU); Newson, Colin, Niddrie, VIC 3042 (AU)
(74) Representative: Plougmann & Vingtoft A/S
(86) International application number: PCT/AU2006/001967
(87) International publication number: WO 2007/070963

(56) References cited:
- EP-A- 1 312 280
- US-A- 4 281 779
- US-A- 4 848 947
- PATENT ABSTRACTS OF JAPAN & JP 11 342 364 A (PENTEL KK) 14 December 1999
- PATENT ABSTRACTS OF JAPAN & JP 11 128 820 A (PENTEL KK) 18 May 1999
- PATENT ABSTRACTS OF JAPAN & JP 11 244 769 A (PENTEL KK) 14 September 1999
- PATENT ABSTRACTS OF JAPAN & JP 11 319 661 A (KOWA SPINNING CO LTD) 24 November 1999

## Description

### Cross-Reference to Related Applications

The present application claims priority from Australian Provisional Patent Application No 2005907296 filed on 23 December 2005, and Australian Provisional Patent Application No 2006902974 filed on 1 June 2006, the contents each of which are incorporated herein by reference.

### Technical Field

This invention relates to a liquid dispensing applicator, and in particular, a liquid dispensing applicator for dispensation of polyvinyl chloride (PVC) priming fluids.

### Background Art

Industrial liquids, in particular volatile liquids, are used in a variety of industries. For example clear PVC priming fluid is commonly used on surfaces of outdoor furniture and swimming pools. Plumbers also use the fluid to clean and prepare PVC piping. The fluid removes contaminants from the surface of the piping so that a solvent cement can be applied and sections of PVC piping joined.

Depending on the constituents, industrial liquids are typically sold in containers. Industrial liquids such as priming fluid can be discomforting to a person's eyes if exposure is prolonged. Moreover, if the fluid comes into contact with a person's skin, irritation of the skin can result. This is particularly the case when the fluid is poured from the container in which it is stored onto a cloth in order to prime surfaces of the pipe.

### Disclosure of Invention

The invention is a liquid dispensing applicator for a volatile liquid, the liquid dispensing applicator comprising:
a cap member for attachment to a hollow bodied container having a reservoir for retaining a liquid, the cap member comprising a sleeve to receive and conduit liquid contained within the reservoir;
a valve member retained within the sleeve of the cap comprising a substantially flexible shaft having a first end projecting from said sleeve exterior; and
a dispensing member co-operating with at least the first end of the shaft;
wherein the valve member is flexibly operable against a bias to move from a first substantially closed position to a second open position to allow displacement of a quantity of liquid from within the sleeve, onto the dispensing member, for subsequent application.

Preferably the dispensing member is absorbent. The dispensing member may comprise a porous, or sponge like, material. The dispensing member may comprise a woven fibre such as felt which may be moulded under pressure to form any desired shape. The dispensing member may be formed by compression-molding a loose fiber piece such as a fiber web. Optionally, or in addition, the dispensing member may comprise a composite, or layered material. The dispensing member may substantially surround the portion of the shaft projecting from said sleeve exterior. The dispensing member additionally surrounds at least a portion of the sleeve.

The dispensing member may be adhered in any fashion to the portion of the shaft projecting from said sleeve exterior or the portion of the shaft projecting from said sleeve exterior and sleeve. For instance the dispensing member may be adhered to the portion of the shaft projecting from said sleeve exterior or the portion of the shaft projecting from said sleeve exterior and sleeve by way of staples, glue, nails or tacks. Optionally exterior surfaces of the portion of the shaft projecting from said sleeve exterior and sleeve may be barbed and the dispensing member may be fixedly attached to the barbs.

The cap member may be removably attachable to a container. The cap member may include an internal thread to attach to a neck of a hollow bodied container. Optionally, the cap member may snap fit to a neck of a hollow bodied container.

The valve member is preferably manufactured from rubber or a flexible polyurethane material.

The valve member may further comprises a retaining member to retain a second end of the substantially flexible shaft in position relative to the sleeve.

The shaft may be hollow to receive and conduit liquid contained within the sleeve and a slit may be provided in a portion of a wall of the first end of the shaft such that when the valve member is flexibly operated against the bias to move from a first substantially closed position to a second open position a quantity of liquid is displaced from within the first end of the shaft and from the slit, onto the dispensing member.

Optionally, the valve member may include a spring wound around the shaft.

In any of the above embodiments, a lock mechanism may be provided to prevent unintentional displacement of the valve member to the second open position. The lock mechanism may include, but not be limited to, a valve positioned within the shaft and may include means external to the shaft to open and close the valve. Optionally a substantially rigid cap may be provided to slide over and substantially surround the dispensing member. The cap may have an additional advantage of substantially preventing the dispensing member from drying out after use.

In any of the above embodiments, the liquid dispensing applicator may further comprise a container having a reservoir for retaining a volatile liquid, where the cap member is attached to the neck of the container.

### Brief Description of Drawings

Embodiments of the invention are now described by way of example with reference to the accompanying drawings in which:-
Fig. 1 shows a perspective view of a first embodiment of a liquid dispensing applicator in a first substantially closed position;
Fig. 2 shows a sectional view of a portion of liquid dispensing applicator shown in figure 1;
Fig. 3 shows a perspective view of the liquid dispensing applicator shown in figure 2 in a second open position; and
Fig. 4 shows a schematic illustration of a second embodiment of a liquid dispensing applicator.

### Best Mode for Carrying Out the Invention

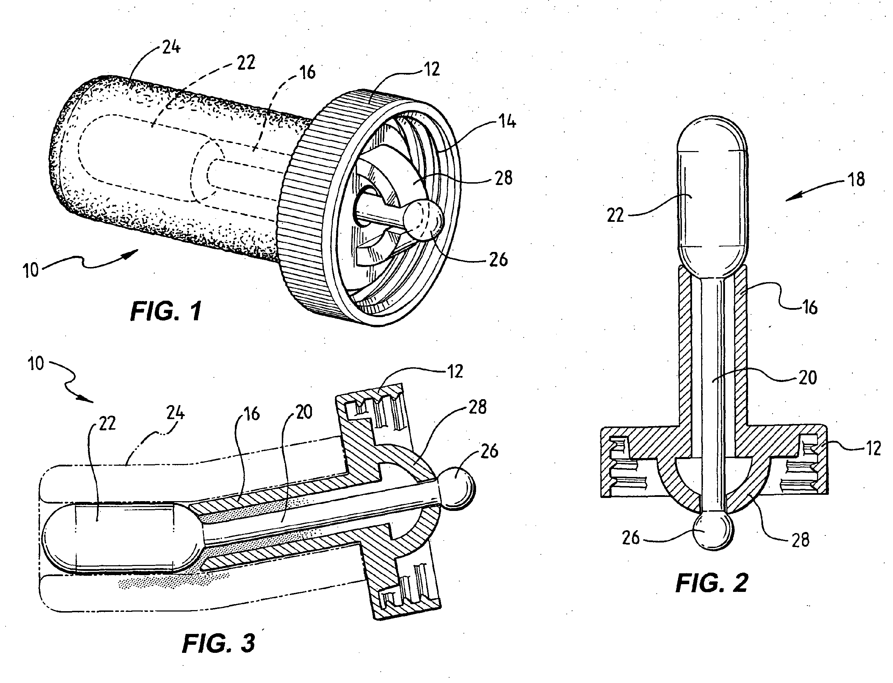

Referring initially to Fig. 1 of the drawings, reference numeral 10 generally designates a liquid dispensing applicator, in accordance with a first embodiment of the invention. The liquid dispensing applicator 10 includes a cap member 12. The cap member 12 includes an internal thread 14 to enable it to attach to a neck of a hollow bodied container having a reservoir for retaining a liquid. The cap member 12 includes a sleeve 16 to receive and conduit liquid contained within the reservoir of the hollow bodied container. A valve member 18 (Fig. 2) is retained within the sleeve 16 of the cap member 12. The valve member 18 is manufactured from a flexible polyurethane material. The valve member 18 includes a substantially flexible shaft 20 having a first end 22 projecting from said sleeve exterior 16.

A dispensing member 24 manufactured from a pad of felt co-operates with the first end 22 of the shaft and additionally surrounds the sleeve 16. The opposing end 26 of the valve member 18 is held in position by way of a retaining member 28 which is molded to a portion of the interior of the cap 12. The positioning of the retaining member 28 does not prevent fluid from passing from the reservoir to the sleeve 16 interior. The valve member 18 is flexibly operable against a bias at said first end to move from a first substantially closed position (Fig. 2) to a second open position (Fig. 3) to allow displacement of a quantity of liquid from within the sleeve 16, onto the dispensing member 24, for subsequent application.

PVC priming fluid is a ketone based solvent and is highly flammable. It is typically sold in 250ml, 500ml or 1 L screw top plastic containers and therefore can be held in the hand of a user. It is envisioned that the cap member will be manufactured from the same material which the containers which hold the PVC priming fluid are manufactured from. However it should be appreciated that the invention is not limited to the application of PVC priming fluid and is suitable for the application of any volatile fluid. Therefore the material from which the cap and valve member are manufactured from will change accordingly.

It is envisioned that the liquid dispensing applicator will be sold separately, or in conjunction with a container of PVC priming fluid. In use the cap of the container of PVC priming fluid is replaced with the cap member 12 of the liquid dispensing applicator 10. The container (not shown) to which the cap member is attached can be easily held by a user and manipulated to bring the dispensing member 24 into contact with a surface onto which the fluid is to be applied. The applicator 10 is tilted so that fluid enters the sleeve 16. Pressure is then applied against the dispensing member 24 such that the shaft 20 flexes against a bias to enable fluid to flow from within the shaft 16 and onto the dispensing member 24. By rolling the applicator 10 from side to side the fluid is able to be dispersed throughout the dispensing member 24, and in turn dispersed over the surface to which the fluid is to be applied.

It is envisioned that the dispensing applicator is for single use, however depending on the material from which the dispensing member 24 is manufactured from the liquid dispensing applicator 10 may be used several times.

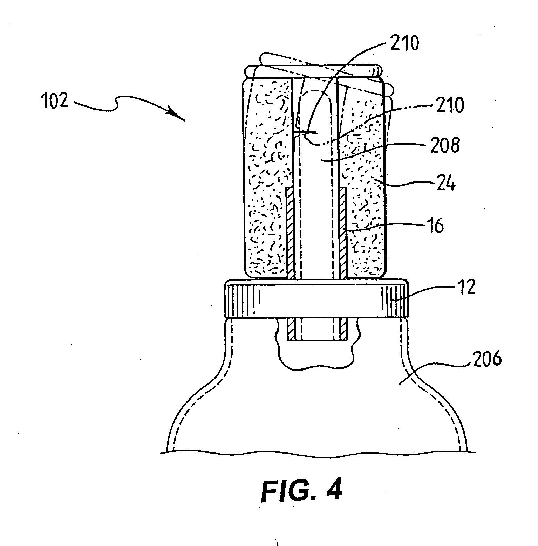

Referring now to figure 4, a liquid dispensing applicator 102 of a second embodiment is illustrated. Like numerals refer to like parts previously described unless otherwise specified. In this embodiment, the cap member 12 includes a sleeve 16 to receive and conduit liquid contained within the reservoir of the hollow bodied container 206. Shaft 208 having a first end projects from said sleeve exterior 16 and comprises a hollow core through which liquid can travel. The shaft is surrounded by a wall manufactured from an elastic material. A slit 210 is provided in a portion of the wall of the shaft 208 and extends through to the core. A dispensing member 24 surrounds the shaft 208. In use, the applicator 102 is tilted so that fluid passes through the core of the shaft 208. Pressure is then applied against the shaft 208 such that the shaft flexes, causing the slit 210 to open which enables liquid to flow from within the core of the shaft 208 and onto the dispensing member 24.

Advantageously, the liquid dispensing applicator in accordance with any of the above embodiments can be easily fitted to a container of PVC priming fluid. The use of the liquid dispensing applicator in accordance with embodiments of the invention, reduces the likelihood of spillage and minimises contact against the user's skin. As a result, less fluid is required to be used relative to traditional techniques.

It should be appreciated that the invention is not limited to the application of priming fluids, and could be used to apply a wide range of liquids, regardless of their classification.

It will be appreciated by persons skilled in the art that numerous variations and/or modifications may be made to the invention as shown in the specific embodiments without departing from the scope of the invention as broadly described. The present embodiments are, therefore, to be considered in all respects as illustrative and not restrictive.

## Claims

1. A liquid dispensing applicator for a volatile liquid, the liquid dispensing applicator comprising:
a cap member 12 for attachment to a hollow bodied container having a reservoir for retaining a liquid, the cap member comprising a sleeve 16 to receive and conduit liquid contained within the reservoir;
a valve member 18 retained within the sleeve 16 of the cap comprising a substantially flexible shaft 20,208 having a first end 22 projecting from said sleeve exterior; and
a dispensing member 24 co-operating with at least the first end 22 of the shaft;
wherein the valve member 18 is flexibly operable against a bias to move from a first substantially closed position to a second open position to allow displacement of a quantity of liquid from within the sleeve, 16 onto the dispensing member, 24 for subsequent application.

2. A liquid dispensing applicator according to claim 1, where the valve member 18 further comprises a retaining member 28 to retain a second end 26 of the substantially flexible shaft in position relative to the sleeve.

3. A liquid dispensing applicator according to claim 1 or 2, where the shaft 208 is hollow to receive and conduit liquid contained within the sleeve and a slit 210 is provided in a portion of a wall of the first end of the shaft.

4. A liquid dispensing applicator according claim 1, where the valve member 18 comprises a spring wound around the shaft.

5. A liquid dispensing applicator according any one of the preceding claims, where the cap member 12 is removably attachable to a container.

6. A liquid dispensing applicator according to claim 5, where the cap member 12 includes an internal thread to attach to a neck of the container.

7. A liquid dispensing applic tor according to according any one of the preceding claims, where the valve member 18 is manufactured from one of a rubber and a flexible polyurethane material.

8. A liquid dispensing applicator according to any one of the preceding claims, further comprising a lock mechanism to prevent unintentional displacement of the valve member from the first closed position to the second open position.

9. A liquid dispensing applicator according to any one of claims 1 to 7, further comprising a substantially rigid cap to removably surround the dispensing member.

10. A liquid dispensing applicator according to any one of the preceding claims, where the dispensing member 24 is manufactured from an absorbent material.

11. A liquid dispensing applicator according to claim 10, where the absorbent member is a felt pad or comprises a composite material.

12. A liquid dispensing applicator according any one of the preceding claims, where the dispensing member 24 substantially surrounds the portion of the shaft projecting from said sleeve exterior and at least a portion of an exterior of the sleeve 16.

13. A liquid dispensing applicator according to any one of the preceding claims, where exterior surfaces of the portion of the shaft projecting from said sleeve exterior and the sleeve exterior include a plurality of barbs and the dispensing member is fixedly attached to the barbs.

## Patentansprüche

1. Flüssigkeitsabgabeapplikator für eine leicht flüchtige Flüssigkeit, wobei der Flüssigkeitsabgabeapplikator umfasst:
ein Kappenelement 12 zum Befestigen an einem Hohlkörperbehälter mit einem Reservoir zum Halten einer Flüssigkeit, wobei das Kappenelement eine Muffe 16 zum Aufnehmen und Leiten von im Behälter enthaltender Flüssigkeit umfasst
ein Ventilelement 18, das in der Muffe 16 der Kappe gehalten wird,
umfassend eine im Wesentlichen flexible Stange 20, 208 mit einem ersten Ende 22, das aus der Muffenaußenseite ragt; und
ein Abgabeelement 24, das mit mindestens dem ersten Ende 22 der Stange
zusammenwirkt;
wobei das Ventilelement flexibel gegen eine Vorspannung bedienbar ist, um sich von einer ersten im Wesentlichen geschlossenen Stellung in eine zweite offene Stellung zu bewegen, um eine Verschiebung einer Menge Flüssigkeit aus dem Inneren der Muffe 16 auf das Abgabeelement 24 für ein anschließendes Auftragen zu ermöglichen.

2. Flüssigkeitsabgabeapplikator nach Anspruch 1, wobei das Ventilelement 18 weiterhin ein Halteelement 28 zum Halten eines zweiten Endes 26 der im Wesentlichen flexiblen Stange in einer Position in Bezug auf die Muffe umfasst.

3. Flüssigkeitsabgabeapplikator nach Anspruch 1 oder 2, wobei die Stange 208 hohl ist, um in der Muffe enthaltende Flüssigkeit aufzunehmen und zu leiten, und ein Schlitz 210 in einem Abschnitt einer Wand des ersten Endes der Stange vorgesehen ist.

4. Flüssigkeitsabgabeapplikator nach Anspruch 1, wobei das Ventilelement 18 eine um die Stange gewickelte Feder umfasst.

5. Flüssigkeitsabgabeapplikator nach einem der vorhergehenden Ansprüche, wobei das Kappenelement 12 abnehmbar am Behälter befestigt ist.

6. Flüssigkeitsabgabeapplikator nach Anspruch 5, wobei das Kappenelement 12 ein Innengewinde zum Befestigen an einem Hals des Behälters umfasst.

7. Flüssigkeitsabgabeapplikator nach einem der vorhergehenden Ansprüche, wobei das Ventilelement 18 aus Gummi oder einem flexiblen Polyurethanmaterial hergestellt ist.

8. Flüssigkeitsabgabeapplikator nach einem der vorhergehenden Ansprüche, weiterhin umfassend einen Sperrmechanismus zum Verhindern eines ungewollten Verschiebens des Ventilelements aus der ersten geschlossenen Position in die zweite offene Position.

9. Flüssigkeitsabgabeapplikator nach einem der Ansprüche 1 bis 7, weiterhin umfassend eine im Wesentlichen starre Kappe, die das Abgabeelement abnehmbar umgibt.

10. Flüssigkeitsabgabeapplikator nach einem der vorhergehenden Ansprüche, wobei das Abgabeelement 24 aus einem absorbierenden Material hergestellt ist.

11. Flüssigkeitsabgabeapplikator nach Anspruch 10, wobei das absorbierende Material ein Filzkissen ist und ein Verbundmaterial umfasst.

12. Flüssigkeitsabgabeapplikator nach einem der vorhergehenden Ansprüche, wobei das Abgabeelement 24 im Wesentlichen den Abschnitt der Stange, der aus der Muffenaußenseite ragt, und mindestens einen Teil einer Außenseite der Muffe 16 umgibt.

13. Flüssigkeitsabgabeapplikator nach einem der vorhergehenden Ansprüche, wobei die Außenflächen des Abschnitts der Stange, der aus der Muffenaußenseite ragt, und die Muffenaußenseite eine Mehrzahl an Widerhaken umfassen und das Abgabeelement fest an diesen Widerhaken befestigt ist.

## Revendications

1. Applicateur distributeur de liquide pour un liquide volatil, l'applicateur distributeur de liquide comprenant :
un élément bouchon (12) à fixer sur un récipient à corps creux muni d'un réservoir pour retenir un liquide, l'élément bouchon comprenant une gaine (16) destinée à recevoir et à conduire du liquide contenu dans le réservoir ;
un élément valve (18) maintenu à l'intérieur de la gaine (16) du bouchon comprenant une tige essentiellement souple (20, 208) dont une première extrémité (22) dépasse de l'extérieur de ladite gaine ; et
un élément distributeur (24) fonctionnant avec au moins la première
extrémité (22) de la tige ;
dans lequel l'élément valve (18) peut être manoeuvré de manière souple en biais pour passer d'une première position essentiellement fermée à une deuxième position ouverte afin de permettre le déplacement d'une quantité de liquide depuis l'intérieur de la gaine (16), sur l'élément distributeur (24), pour application ultérieure.

2. Applicateur distributeur de liquide selon la revendication 1, dans lequel l'élément valve (18) comprend également un élément de retenue (28) destiné à retenir une deuxième extrémité (26) de la tige essentiellement souple dans sa position par rapport à la gaine.

3. Applicateur distributeur de liquide selon la revendication 1 ou 2, dans lequel la tige (208) est creuse de manière à recevoir et à conduire du liquide contenu dans la gaine et une fente (210) est ménagée dans une partie d'une paroi de la première extrémité de la tige.

4. Applicateur distributeur de liquide selon la revendication 1, dans lequel l'élément valve (18) comprend un ressort enroulé autour de la tige.

5. Applicateur distributeur de liquide selon l'une quelconque des revendications précédentes, dans lequel l'élément bouchon (12) est fixé sur un récipient de manière amovible.

6. Applicateur distributeur de liquide selon la revendication 5, dans lequel l'élément bouchon (12) comporte un filetage interne permettant de le fixer sur un goulot du récipient.

7. Applicateur distributeur de liquide selon l'une quelconque des revendications précédentes, dans lequel l'élément valve (18) est fabriqué dans un matériau en caoutchouc ou un matériau en polyuréthane souple.

8. Applicateur distributeur de liquide selon l'une quelconque des revendications précédentes, comprenant également un mécanisme de verrouillage destiné à empêcher le déplacement involontaire de l'élément valve de la première position fermée à la deuxième position ouverte.

9. Applicateur distributeur de liquide selon l'une quelconque des revendications 1 à 7, comprenant également un bouchon essentiellement rigide pour entourer de manière amovible l'élément distributeur.

10. Applicateur distributeur de liquide selon l'une quelconque des revendications précédentes, dans lequel l'élément distributeur (24) est fabriqué dans un matériau absorbant.

11. Applicateur distributeur de liquide selon la revendication 10, dans lequel l'élément absorbant est un tampon en feutre ou comprend un matériau composite.

12. Applicateur distributeur de liquide selon l'une quelconque des revendications précédentes, dans lequel l'élément distributeur (24) entoure essentiellement la partie de la tige dépassant de l'extérieur de ladite gaine et au moins une partie de l'extérieur de la gaine (16).

13. Applicateur distributeur de liquide selon l'une quelconque des revendications précédentes, dans lequel les surfaces extérieures de la partie de la tige dépassant de l'extérieur de ladite gaine et l'extérieur de la gaine comportent plusieurs picots et l'élément distributeur est fixé sur les picots de manière fixe.
